# EUROPEAN PATENT SPECIFICATION

(11) **EP 1 443 856 B1**
(45) Date of publication and mention of the grant of the patent: **01.02.2006**
(21) Application number: 02774976.1
(22) Date of filing: 04.11.2002
(51) Int. Cl.: A61B 5/026, A61B 8/06

(54) **BLOOD FLOW VELOCITY MEASUREMENT**
MESSUNG DER BLUTFLUSSGESCHWINDIGKEIT
MESURE DE LA VITESSE D'UN FLUX

(30) Priority: 07.11.2001 GB 0126804
(43) Date of publication of application: 11.08.2004
(73) Proprietor: UNIVERSITY COLLEGE LONDON, London WC1E 6BT (GB)
(72) Inventor: BEARD, Paul, London W2 4NY (GB)
(74) Representative: Greene, Simon Kenneth
(86) International application number: PCT/GB2002/004977
(87) International publication number: WO 2003/039364

(56) References cited:
- EP-A- 1 134 559
- WO-A-00/00783
- WO-A-98/38904
- US-A- 6 100 969
- US-A- 6 161 426
- BEARD P C ET AL: "Optical fiber photoacoustic-photothermal probe" OPTICS LETTERS, 1 AUG. 1998, OPT. SOC. AMERICA, USA, vol. 23, no. 15, pages 1235-1237, XP002248562 ISSN: 0146-9592
- BLODGETT D W ET AL: "LASER ULTRASONIC TECHNIQUES FOR ASSESSMENT OF TOOTH STRUCTURE" PROCEEDINGS OF THE SPIE, SPIE, BELLINGHAM, VA, US, vol. 3914, 22 January 2000 (2000-01-22), pages 588-598, XP008003906

## Description

The invention relates to a method and apparatus for measuring the speed or velocity of fluid in tubes, especially blood in the human or animal body, and in particular to a method and apparatus using a so-called "Doppler-shift" signal.

To measure blood flow using a Doppler technique, sound, generally ultrasound, is transmitted into the human or animal body, and scattered by red blood cells moving in blood vessels. A sound receiver is positioned to receive the scattered sound. If the red blood cells scattering sound back towards the receiver are moving towards the receiver, the frequency of the received scattered sound is higher than that of the transmitted sound. Conversely, if the red blood cells scattering sound back towards the receiver are moving away from the receiver, the frequency of the received scattered sound is lower than that of the transmitted sound. By measuring the frequency of the received sound and comparing it to the frequency of the transmitted sound, the speed of blood flow can be measured.

A simple, continuous flow Doppler system will now be described. An imaging head has a transmission ultrasound transducer for emitting ultrasound and a reception ultrasound transducer 6. The imaging head is shown adjacent to a blood vessel 8 filled with moving red blood cells 10. The sound emitter transmits a sound beam towards the blood vessel 8, and the blood cells 10 scatter sound back to the microphone 6.

The electronics include an oscillator, which drives a transmission amplifier. The received sound is amplified by amplifier, and compared with the original signal in demodulator 22. An output is provided, for example to audio headphones 24.

Signal processing to extract the Doppler signal from other reflected waves, noise, and the like is required. The blood vessel 8 is enclosed within human tissue 26, and much of the reflected sound signal received in ultrasound transducer 6 is scattered by stationary tissue and not the red blood cells 10. Therefore, the amount of signal in the received signal is normally much less than the noise. One approach to signal processing is to carry out real time spectral analysis.

Further complications arise from the fact that blood vessels 8 are not straight, and that that the human body contains many blood vessels 8. The continuous system is essentially unable to resolve different depths, which makes it very difficult to separate out signals from different blood vessels 8.

An alternative signal processing scheme implements a pulsed wave flow detector, which is capable of resolving blood flow at different depths. The sensor head 2 includes a single transducer. An ultrasound pulse is emitted by the ultrasound transducer, and the reflection is picked up a little later by the same transducer. The demodulator 22 compares the reflection signal received in a time window with the emitted pulse to determine the frequency shift. The time window is centred a variable time delay after the emitted pulse is emitted and this time delay corresponds to the distance from the transducer to the blood vessel 8. Accordingly, by varying the time delay from the emission of the pulse to the time window, the flow velocity of blood can be determined at various depths within the body.

However, both of these approaches suffer from the low signal to noise ratio, i.e. the small amplitude of the reflected signal from blood cells in comparison with all the other reflected signals emitted from other tissue. One reason for the small amount of reflected signal is the low acoustic mismatch between blood and tissue. This means that only a very small percentage of the signal arriving at the blood vessel is reflected back.

It would accordingly be highly advantageous to provide a technique for measuring flow in which this problem was alleviated.

Moreover, the need to measure flow is not limited to measuring blood flow and it would accordingly also be advantageous to measure flow in other systems.

According to the invention there is provided a flow measuring apparatus according to Claim 1.

Much better signal to noise performance may be achieved than by using prior ultrasound only systems.

The apparatus may be used in particular for measuring the speed of blood flow in tissue. By exciting ultrasound directly in the blood vessel by absorption of laser radiation having components at ultrasound frequency, much larger acoustic signals can be received than using the prior art approach. Ultrasound is excited in the blood vessels much more efficiently than in the prior art since the technique no longer relies on sound reflected in the blood vessels, which in view of the low acoustic mismatch between different tissue is only a small fraction of the input ultrasound. Instead, ultrasound is generated by the excitation laser light which is strongly absorbed in haemoglobin at visible and near infra-red wavelengths.

Since the optical absorption of haemoglobin is strong the technique selectively measures the speed in blood vessels, where required, rather than detecting signals from other tissue. In preferred embodiments of the invention, the amount of absorption is determined as well as the frequency shift. This enables the degree of oxygenation in the blood vessel to be measured at the same time as this is related to the absorption in blood.

In one approach the modulator is arranged to emit a series of tonebursts of light modulated at an ultrasound frequency and the demodulator is arranged to determine the change in the time shift in the received ultrasound signals excited by different tonebursts to determine flow speed. The tonebursts may each include a sequence of short pulses of light with a time separation of 0.02 microseconds to 1 microsecond. Using this approach, relatively conventional so-called Doppler processing may be carried out.

In an alternative approach the modulator is arranged to emit a series of bursts of light and the demodulator is arranged to determine the time shift between the received ultrasound signals excited by successive bursts. The bursts may be single pulses of light. This latter approach requires the modulation to only be short single pulses which may be conveniently generated at higher power, for example by using a Q-switched laser. This higher power may make it easier to generate sufficient ultrasound for detection. It should be noted that a single short delta-like pulse has a significant frequency component in the ultrasound range.

The ultrasound detector may be a directional detector for picking up incident ultrasound from a predetermined direction.

In alternative arrangements, the ultrasound detector may be a detector array. Processing means may be provided for synthesising from the signals received from the detector array the signals received from at least one location within the sample and determining the speed of flow from the synthesised signals. The processing means may be implemented in the demodulator, or separately.

Embodiments of the invention use a transparent polymer film Fabry-Perot interferometer, a continuous wave interrogation laser source for supplying a continuous light signal to the transparent Fabry-Perot interferometer and an optical detector for detecting light from the interrogation laser source reflected and interfered in the interferometer. These components constitute a suitable ultrasound detector for use in the invention.

Alternative embodiments use a piezoelectric ultrasound detector.

The detector may be a single detector or an array of detectors.

Embodiments of the invention provide an optical head having a housing, optical inputs for the excitation laser signals and the continuous light signal, and partially reflective mirrors for directing the continuous light signal and the excitation laser signal.

The excitation laser may be a Q-switched laser and the modulator may be a circuit arranged to output pulses with a time separation 0.02 microseconds to 1 microsecond to drive the Q-switched laser. The inverse of the period between adjacent electronic pulses, the duration of the pulses and the PRR all contribute to the ultrasound frequency components that are generated.

Alternative embodiments of the invention may use an electro-optic modulator to modulate a continuous wave laser to provide similar ultrasound frequency components.

Another aspect, which does not constitute a portion of the invention, relates to a photoacoustic measuring head, comprising: a housing; a transparent Fabry-Perot polymer film interferometer; an input for excitation laser light modulated at an ultrasound frequency; an input for a continuous wave interrogation light beam; and partially reflective mirrors for directing the continuous light signal and the excitation laser signal from their reflective optical inputs normally onto the Fabry-Perot interferometer.

A method of measuring fluid flow in a sample defining a flow path includes: modulating an excitation laser at an ultrasound frequency; directing the excitation laser output into the sample to excite ultrasound in fluid in the flow path; receiving ultrasound excited in the sample in a detector; and comparing the received ultrasound with the ultrasound frequency modulating the pulse train to determine the speed of flow in the flow path in the irradiated sample.

Preferably, the excitation laser emits a pulse train of predetermined length, and the frequency, phase or time delay of received ultrasound is compared with the modulation frequency for sound waves received in a given time window after the pulse train is emitted. The time window may be varied in order to vary the depth within the sample at which flow is measured. ultrasound frequency modulating the pulse train to determine the speed of flow in the flow path in the irradiated sample.

Preferably, the excitation laser emits a pulse train of predetermined length, and the frequency, phase or time delay of received ultrasound is compared with the modulation frequency for sound waves received in a given time window after the pulse train is emitted. The time window may be varied in order to vary the depth within the sample at which flow is measured.

The method may advantageously be used for measuring blood flow in human or animal tissue.

For a better understanding of the invention, embodiments will now be described, purely by way of example, with reference to the accompanying drawings in which:
Figure 1 shows a schematic diagram of an embodiment of a blood flow measuring apparatus according to the invention;
Figure 2 shows a variation of the embodiment of Figure 1;
Figure 3 illustrates a laser modulation signal used in the invention;
Figure 4 illustrates a further embodiment of a blood flow measuring apparatus according to the invention and
Figure 5 shows in more detail a sensor head in accordance with the invention.

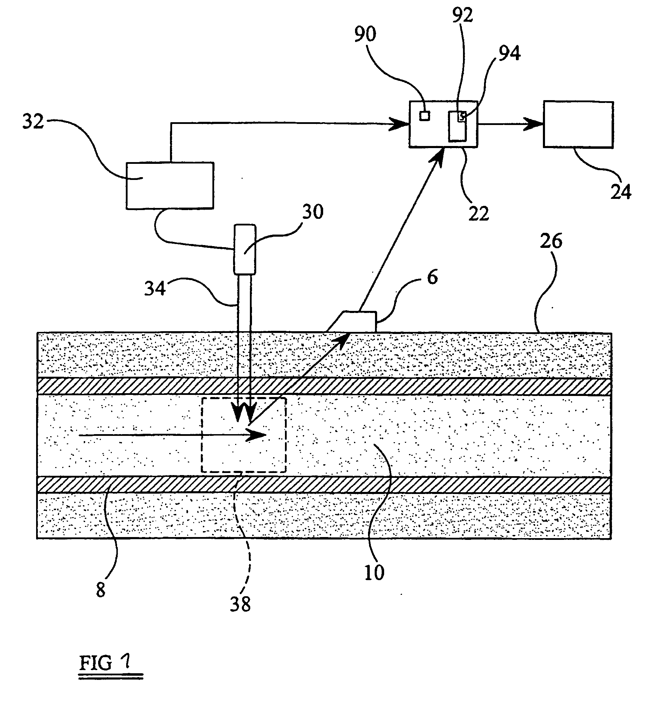

Referring to Figure 1, blood flow measuring apparatus includes a laser 30 driven by modulator 32 and sensor 6. The modulator 32 is connected to control the laser 30 and a demodulator 22 is connected with inputs from the sensor 6 and modulator 32 and an output connected to an output device. The demodulator 22 may include a processor 90 and a memory 92, the latter containing code 94 for causing the processor 90 to carry out the required processing steps to demodulate the data and provide a suitable output.

The output device may be a computer, data store, headphones, graphical readout, or a combination of any or all of these or other suitable data output devices.

The laser may be a high repetition rate (e.g. 5 MHz) Q-switched laser, or even a conventional laser diode.

The sensor 6 is directional, that is to say of a type that is only sensitive to ultrasound signals coming substantially in one direction, illustrated by the arrow in Figure 1. The sensor 6 may be any suitable ultrasonic sensor, for example a piezoelectric detector or a Fabry- Perot polymer film sensor of the type that will be described later. The size of the sensor 6 in this embodiment which uses a directional sensor is greater than the wavelength of ultrasound.

In use, the laser 30 produces laser light 34 which is directed through human tissue 26 to illuminate a region 38 of blood vessel 8. The light in the illuminated region 38 excites ultrasound in red blood cells 10. The laser light 34 is modulated to have components at an ultrasound frequency. As will be appreciated, this may be done in a number of ways. In a preferred embodiment of invention, the laser 30 is a Q-switched laser modulated by the modulator 32 to produce pulse trains with a period between pulses of from 0.02 µs to 1 µs, preferably 0.1 to 0.4 µs. Each individual pulse may be, for example, 10ns long.

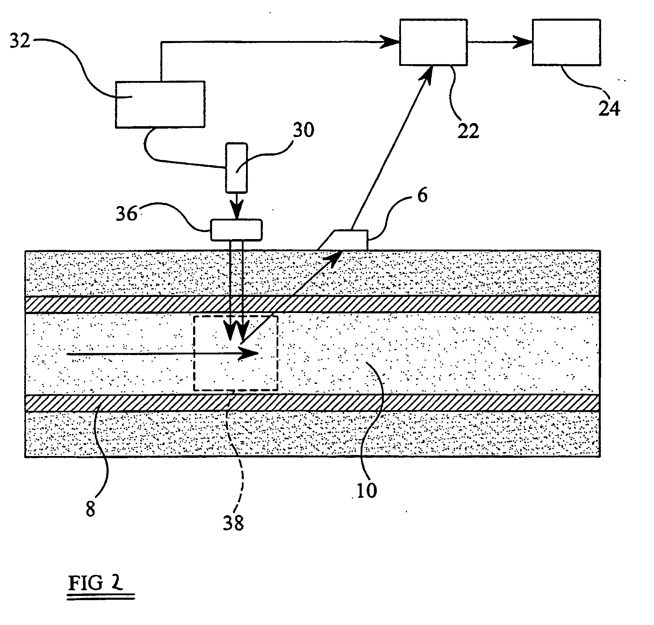

In an alternative embodiment illustrated in Figure 2, an electro-optic modulator 36 may be placed in front of laser 30 to modulate the laser beam 34 at ultrasound frequency

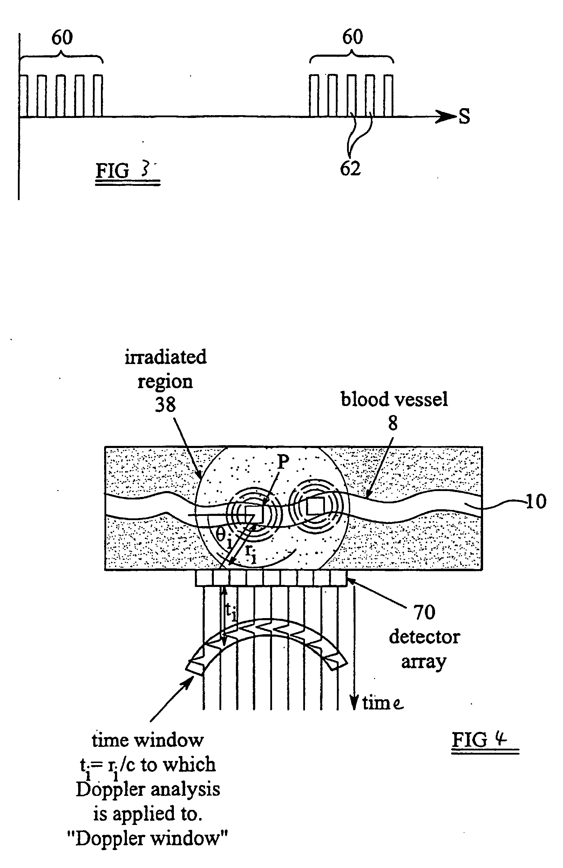

Figure 3 shows an example of a modulation applied to the laser signal. A tone burst 60 includes a pulse train with a number of individual pulses 62, each having a pulse length, the individual pulses being separated by a pulse time delay. The ultrasound modulation frequency is the inverse of the pulse time delay. The inverse of the time between successive tone burst is the pulse train repetition rate.

The modulation frequency affects velocity resolution since a higher modulation frequency results in a higher velocity-induced Doppler shift, i.e. a larger frequency difference between modulation frequency and received frequency. However, frequency-dependent acoustic attenuation in tissue limits the maximum modulation frequency that may be used. For measurement down to millimetre scale in tissue, a frequency of up to 20 MHz might be acceptable, but for centimetre penetration depths the frequency should be of the order of a few MHz. For a frequency in the range 1 to 10 MHz and as the measured velocity component varies from 0 to 1 metres per second, the Doppler change in frequency varies typically in the range 0 to 6 kHz.

The length of each tone burst, i.e. the time during which the modulated laser pulse is emitted, needs to be long to improve frequency resolution, but short to improve spatial resolution. Reducing the number of cycles of modulation frequency within the pulse train broadens the spectrum associated with the sinusoid spectral leakage. For millimetre resolution at centimetre ranges a tone burst length of 0.1 to 1 microsecond is appropriate. For millimetre ranges, i.e. less than 1 centimetre, the modulation frequency may be increased enabling the tone burst width to be reduced without incurring spectral leakage.

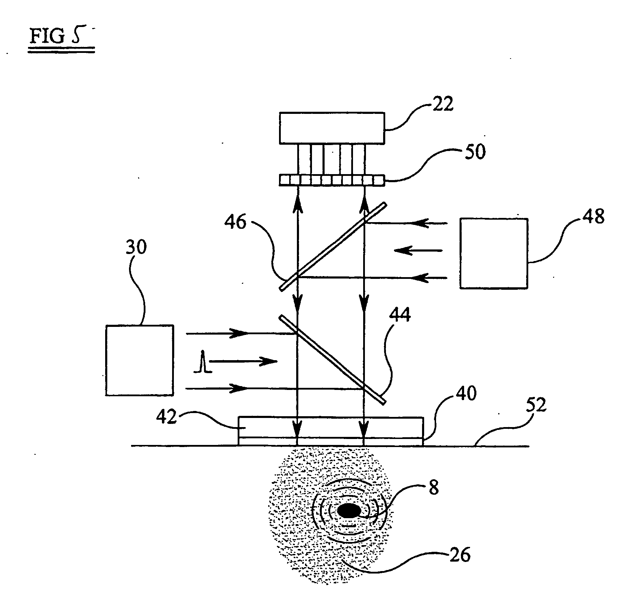

Although the tone burst may be a "pulse train", the invention is not limited to simple pulses and a short tone burst having a sinusoid or other shaped tone of predetermined modulation frequency and length may be provided, for example using an electro-optic modulator 36 (Fig. 4) to generate an arbitrary wave form together with a high power continuous wave laser.

However, the arrangement of Figure 3 using either a high repetition rate (e.g. 5 MHz) Q-switch laser or direct modulation of a laser diode uses less expensive components. The wave train is emitted as above but the signal burst comprises pulses of order 10 nanoseconds separated by 0.2 microseconds rather than a sinusoid as above.

In both arrangements illustrated in Figures 3 and 4 the blood cells 10 absorb the laser light preferentially to the surrounding tissue. The blood cells 10 are caused thereby to emit ultrasound. The individual pulse duration, repetition rate and number of pulses in the modulation signal 34 all contribute to the components of the ultrasound generated.

However, the arrangement of Figure 1 using either a high repetition rate (e.g. 5 MHz) Q-switch laser or direct modulation of a laser diode uses less expensive components. The wave train is emitted as above but the signal burst comprises pulses of order 10 nanoseconds separated by 0.2 microseconds rather than a sinusoid as above.

In both arrangements illustrated in Figures 1 and 2 the blood cells 10 absorb the laser light preferentially to the surrounding tissue. The blood cells 10 are caused thereby to emit ultrasound. The individual pulse duration, repetition rate and number of pulses in the modulation signal 34 all contribute to the components of the ultrasound generated.

This technique of generating ultrasound by light may be referred to as photo-acoustic generation. Unlike conventional Doppler techniques photo-acoustic generation requires the laser energy to be deposited in a time that is short compared to the acoustic transmission time across the illuminated region, i.e. before the stress induced by the laser has a chance to propagate away. This effect means that the modulation frequency may preferably be higher than otherwise, for example 10 MHz plus or minus 5 MHz.

This ultrasound 14 is picked up by the ultrasound transducer 6 which is placed against tissue 26. The transducer 6 is illustrated offset from the illuminated region although this is not essential. The transducer is located such that ultrasound from the blood vessel to the transducer 6 is at an angle θ to the normal to the blood vessel; in general the angle θ may be estimated.

The ultrasound received in a given time window after the pulse train is emitted by laser 6 is processed. The time window is centred on a time delayed from the time the pulse train is emitted by a time calculated from the distance of the transducer 6 from the irradiated region 38 of the blood vessel 8 divided by the speed of ultrasound in tissue. By varying the time delay from emitting the pulse train to the given time window the depth in human tissue at which the blood flow is measured may be varied.

The blood flow velocity is then calculated from the ultrasound frequency, phase and/or time delay received in the time window. The output device 24 then provides information regarding the blood speed. tonebursts. This can be processed, using quadrature phase detection methods (among others), to provide an estimation of the phase difference between the detected signal and the optical excitation. A series of sampled phase values will be obtained as a result of a train of tonebursts. These phase values will oscillate in time at a frequency that happens to be that given by the regular Doppler equation. So although we are not directly measuring the conventional Doppler frequency shift we end up with its numerical value as a consequence of the type of processing employed, namely, phase shift extraction.

This approach requires an appropriate laser modulation such as a repetitive toneburst as would be employed in conventional pulsed Doppler ultrasound using an ultrasound excitation. A suitable modulation is a 5 cycle, 5MHz toneburst, at a repetion rate of 10KHz.

Ideally, the phase shift signal processing method uses a method of generating an arbitrary optical waveform for example by directly modulating the injection current of a laser diode or externally modulating a CW laser. The ability to produce arbitrary excitation waveforms allows other phase shift measurement schemes to be employed such as random, pseudo random and frequency coded excitation.

An alternative approach to carrying out the signal processing will now be described, which may be referred to as a time shift/correlation method.

In this approach the time shift between successive detected signals is obtained directly and used to determine velocity. A repetitive excitation toneburst as described above could be used although an optical excitation comprising a train of individual short delta function-like pulses or bursts of light would be preferable.

To obtain velocity, the time shift between two successive characteristic photoacoustic signals originating from the same moving blood cell cluster is obtained directly by calculating the cross-correlation between the two. Using the speed of sound, the distance the emitter has moved between two successive excitation events is obtained and then using the repetition interval, the flow velocity can be obtained. With this approach, the aliasing and directional ambiguity problems associated with phase shift measurement toneburst as described above could be used although an optical excitation comprising a train of individual short delta function-like pulses or bursts of light would be preferable.

To obtain velocity, the time shift between two successive characteristic photoacoustic signals originating from the same moving blood cell cluster is obtained directly by calculating the cross-correlation between the two. Using the speed of sound, the distance the emitter has moved between two successive excitation events is obtained and then using the repetition interval, the flow velocity can be obtained. With this approach, the aliasing and directional ambiguity problems associated with phase shift measurement techniques are avoided. It is also intrinsically broadband requiring short duration photoacoustic signals. It therefore lends itself naturally to the type of excitation provided by high repetition rate Q switched lasers. These can provide a train of ns pulse at kHz repetition rates with sufficiently high pulse energy.

Note that due to the high density and small size of red blood cells, it is not the photoacoustic signal generated within an individual moving red blood cell that is detected. Rather it is the collective signal produced by a particular moving group of cells which, by providing a unique photoacoustic signature, can be detected and tracked as is moves along the vessel. Thus, as with conventional pulsed Doppler ultrasound, the technique is contingent upon a non-uniform red cell density distribution.

The time-shift measurement method may alleviate any difficulties of providing sufficient signal power in tonebursts to generate detectable photoacoustic signals.

The skilled person will be aware of many suitable signal processing approaches from conventional Doppler ultrasound measurements. For example frequency modulation may be used using a non-duration frequency chirp pulse to obtain range information. Such approach may also be used in the arrangement of the present invention.

An alterative embodiment of the apparatus according to the invention will now be described with reference to Figure 4, which uses a detector array indicating the direction of flow. θᵢ is the angle between this vector and the line between P and a specific detector element i.

To calculate the doppler shift a temporal window is applied to the time record of each detector element i with the position of the window corresponding to the distance from P to the element divided by the speed of sound. From each of the windowed signals, the Doppler shift dfᵢ is extracted, weighted by cos θᵢ (which.will be different for each element eg zero for θ = 90° increasing to a maximum for θ = 0°), and summed over all i to produce an average Doppler shift.

The weighted-averaging process is important because in principle there could be another source (an adjacent red cell or cluster of cells or perhaps another vessel) located such that it also provides a signal that arrives within one of the detector element time windows and corrupts the Doppler signal extracted from that element. However simple geometry dictates that it would only coincide with that specific detector element time window - the signals extracted from the remaining elements would not be affected. So the influence on the weighted average Doppler shift would be small, assuming a reasonable number of detectors.

The above description assumes that the Doppler shift is extracted from the same signals that were acquired to form the anatomical image. This would assume the laser excitation pulse parameters were suitable for this - i.e. a train of pulses at a suitable PRR. In practice however, it may be beneficial if the anatomical image is formed with one set of excitation parameters (e.g. discrete pulses) for optimum spatial resolution and then the Doppler laser excitation (e.g. multiple pulses, pseudo-toneburst etc.) applied once the point in the vessel of interest had been identified.

The above discussion is in terms of a single point P. In practice many points or an area might similarly be identified to map flow along or across vessels.

Note that Figure 6 is highly schematic. The laser irradiation geometry may be selected as required - the illumination could be delivered on the opposite side to the detector array (forward-mode) or on the same side anatomical image is formed with one set of excitation parameters (e.g. discrete pulses) for optimum spatial resolution and then the Doppler laser excitation (e.g. multiple pulses, pseudo-toneburst etc.) applied once the point in the vessel of interest had been identified.

The above discussion is in terms of a single point P. In practice many points or an area might similarly be identified to map flow along or across vessels.

Note that Figure 4 is highly schematic. The laser irradiation geometry may be selected as required - the illumination could be delivered on the opposite side to the detector array (forward-mode) or on the same side (backward mode). The latter could be implemented by inserting an acoustically transparent optical reflector between the array and the tissue surface.

Alternatively the Fabry Perot sensing system as discussed below could be employed. The sensor head is transparent so the excitation pulses could be transmitted through it. In general, however the detector array is non-specific - it could be the Fabry Perot sensor, an array of piezoelectric elements or some other transducer array.

Referring to Figure 5, a suitable sensor head is shown. The head includes a transparent Fabry-Perot interferometer 40, mounted on a sensor mounting 42. Wavelength selective mirror 44 directs the excitation laser pulses from laser 30 on to the radiated volume, and wavelength selective mirror 46 directs light from a continuous wave interrogating laser source 48 on to the Fabry-Perot interferometer 40. By using wavelength selective mirrors 46, 44 the light paths of the excitation laser pulses and the interrogating continuous wave light can be correctly directed to and from the transparent interferometer 40 without significantly interfering with one another. The reflected light from the continuous wave interrogating laser 48 on the Fabry-Perot interferometer 40 is reflected onto detector array 50, here an array of photo diodes, for processing in demodulator 22.

The implementation is not limited to the embodiments described above. The skilled person will be aware of many suitable light sources, modulators and detectors that may be used in connection with the invention.

## Claims

1. Flow measuring apparatus, including:
an excitation laser (30) for irradiating a sample defining a tube allowing fluid flow with excitation laser light,
an ultrasound detector (6) for receiving ultrasound excited in the sample by the excitation laser light
a demodulator (22) for determining the speed of fluid flow in the irradiated sample by comparing the frequency, phase and/or timing of the received ultrasound with that of the modulation of the excitation laser light; and
a modulator (32) for modulating the excitation laser light to have a modulation component at an ultrasound frequency,
**characterised by** the modulator (32) being arranged to control the excitation laser to emit a series of tonebursts, each toneburst having a plurality of pulses of light emitted at an ultrasound frequency and
the demodulator (22) being arranged to determine the change in the time shift in the received ultrasound signals excited by different tonebursts to determine flow speed.

2. Flow measuring apparatus according to claim 1 wherein the modulator is arranged to control the excitation laser to emit tonebursts each including a sequence of short pulses of light with a time separation of 0.02 microseconds to 1 microsecond

3. Flow measuring apparatus according to any preceding claim wherein the modulator is a circuit arranged to output electronic pulses and the modulator is connected to the laser to drive the laser.

4. Flow measuring apparatus according to any preceding claim wherein the ultrasound detector (6) is a directional detector for picking up incident ultrasound from a predetermined direction.

5. Flow measuring apparatus according to any of claims 1 to 4 wherein the ultrasound detector (6) is a detector array.

6. Flow measuring apparatus according to claim 5 further including processing means (22) for synthesising from the signals received from the detector array the signals received from at least one location within the sample and determining the speed of blood flow from the synthesised signals.

7. Flow measuring apparatus according to claim 6 wherein the processing means (22) is implemented in the demodulator.

8. Flow measuring apparatus according to any preceding claim wherein the demodulator (22) is a computer having a processor and a memory including code for carrying out the step of determining the speed of blood flow in the irradiated sample by comparing the frequency, phase and/or timing of the received ultrasound with that of the modulation of the excitation laser light.

## Revendications

1. Appareil de mesure de flux, incluant :
un laser d'excitation (30) pour irradier un échantillon définissant un tube permettant un écoulement de fluide, avec une lumière du laser d'excitation,
un détecteur à ultrasons (6) pour recevoir les ultrasons excités dans l'échantillon par la lumière du laser d'excitation,
un démodulateur (22) pour déterminer la vitesse de l'écoulement de fluide dans l'échantillon irradié en comparant la fréquence, la phase et/ou la temporisation des ultrasons reçus avec celles de la modulation de la lumière du laser d'excitation ; et
un modulateur (32) pour moduler la lumière du laser d'excitation de manière à présenter une composante de modulation à une fréquence ultrasonore,
**caractérisé en ce que** le modulateur (32) est agencé pour commander le laser d'excitation pour émettre une série de salves sonores, chaque salve sonore ayant une pluralité d'impulsions de lumière émises et une fréquence ultrasonore, et
le démodulateur (22) est agencé pour déterminer le changement du décalage temporel dans les signaux ultrasonores reçus excités par différentes salves sonores pour déterminer la vitesse d'écoulement.

2. Appareil de mesure de flux selon la revendication 1, dans lequel le modulateur est agencé pour commander le laser d'excitation pour émettre des salves sonores qui incluent chacune une séquence de courtes impulsions de lumière avec une séparation temporelle de 0,02 microsecondes à 1 microseconde.

3. Appareil de mesure de flux selon l'une quelconque des revendications précédentes, dans lequel le modulateur est un circuit agencé pour délivrer des impulsions électroniques, et le modulateur est connecté au laser pour piloter le laser.

4. Appareil de mesure de flux selon l'une quelconque des revendications précédentes, dans lequel le détecteur à ultrasons (6) est un détecteur directionnel pour détecter des ultrasons incidents depuis une direction prédéterminée.

5. Appareil de mesure de flux selon l'une quelconque des revendications 1 à 4, dans lequel le détecteur à ultrasons (6) est un réseau de détecteurs.

6. Appareil de mesure de flux selon la revendication 5, incluant en outre des moyens de traitement (22) pour synthétiser, à partir des signaux reçus depuis le réseau de détecteurs, les signaux reçus depuis au moins un emplacement à l'intérieur de l'échantillon et pour déterminer la vitesse de l'écoulement sanguin à partir des signaux synthétisés.

7. Appareil de mesure de flux selon la revendication 6, dans lequel les moyens de traitement (22) sont mis en oeuvre dans le démodulateur.

8. Appareil de mesure de flux selon l'une quelconque des revendications précédentes, dans lequel le démodulateur (22) est un ordinateur ayant un processeur et une mémoire qui inclut un code pour exécuter l'opération de détermination de la vitesse de l'écoulement sanguin dans l'échantillon irradié en comparant la fréquence, la phase et/ou la temporisation des ultrasons reçus avec celles de la modulation de la lumière du laser d'excitation.

## Patentansprüche

1. Flussmessvorrichtung, die folgende Merkmale umfasst:
einen Erregungslaser (30) zum Bestrahlen einer Probe, die eine Röhre definiert, die einen Fluidfluss mit Erregungslaserlicht ermöglicht,
einen Ultraschalldetektor (6) zum Empfangen von Ultraschall, der in der Probe durch das Erregungslaserlicht erregt wird,
einen Demodulator (22) zum Bestimmen der Geschwindigkeit des Fluidflusses in der bestrahlten Probe durch Vergleichen der Frequenz, Phase und/oder Zeitgebung des empfangenen Ultraschalls mit der der Modulation des Erregungslaserlichts; und
einen Modulator (32) zum Modulieren des Erregungslaserlichts, um eine Modulationskomponente bei einer Ultraschallfrequenz zu haben,
**dadurch gekennzeichnet, dass** der Modulator (32) angeordnet ist, um den Erregungslaser zu steuern, um eine Reihe von Tonimpulsen zu emittieren, wobei jeder Tonimpuls eine Mehrzahl von Lichtpulsen aufweist, die bei einer Ultraschallfrequenz emittiert werden, und
der Demodulator (22) angeordnet ist, um die Änderung bei der Zeitverschiebung bei den empfangenen Ultraschallsignalen zu bestimmen, die durch unterschiedliche Tonimpulse erregt werden, um die Flussgeschwindigkeit zu bestimmen.

2. Flussmessvorrichtung gemäß Anspruch 1, bei der der Modulator angeordnet ist, um den Erregungslaser zu steuern, um Tonimpulse zu emittieren, die jeweils eine Sequenz von kurzen Lichtpulsen umfassen, mit einem Zeitabstand von 0,02 Mikrosekunden bis 1 Mikrosekunde.

3. Flussmessvorrichtung gemäß einem der vorhergehenden Ansprüche, bei der der Modulator eine Schaltung ist, die angeordnet ist, um elektronische Pulse auszugeben, und der Modulator mit dem Laser verbunden ist, um den Laser zu treiben.

4. Flussmessvorrichtung gemäß einem der vorhergehenden Ansprüche, bei der der Ultraschalldetektor (6) ein Richtungsdetektor ist, zum Aufnehmen von einfallendem Ultraschall von einer vorbestimmten Richtung.

5. Flussmessvorrichtung gemäß einem der Ansprüche 1 bis 4, bei der der Ultraschalldetektor (6) ein Detektorarray ist.

6. Flussmessvorrichtung gemäß Anspruch 5, die ferner eine Verarbeitungseinrichtung (22) umfasst, zum Synthetisieren der Signale, die von zumindest einer Stelle in der Probe empfangen werden, von den Signalen, die von dem Detektorarray empfangen werden, und zum Bestimmen der Geschwindigkeit des Blutflusses von den synthetisierten Signalen.

7. Flussmessvorrichtung gemäß Anspruch 6, bei der die Verarbeitungseinrichtung (22) in dem Demodulator implementiert ist.

8. Flussmessvorrichtung gemäß einem der vorhergehenden Ansprüche, bei der der Demodulator (22) ein Computer ist, der einen Prozessor und einen Speicher aufweist, der einen Code zum Ausführen des Schritts des Bestimmens der Geschwindigkeit des Blutflusses in der bestrahlten Probe umfasst, durch Vergleichen der Frequenz, Phase und/oder Zeitgebung des empfangenen Ultraschalls mit der der Modulation des Erregungslaserlichts.
